# EUROPEAN PATENT APPLICATION

(11) **EP 1 561 485 A1**
(43) Date of publication of application: **10.08.2005**
(21) Application number: 05100664.1
(22) Date of filing: 01.02.2005
(51) Int. Cl.: A61M 15/00

(54) **Portable inhaler compression device**

(30) Priority: 03.02.2004 SI 200400039
(71) Applicant: Bremed d.o.o. Breginj, 5223 Breginj (SI)
(72) Inventor: Simac, Branko, SI-5223, Breginj (SI); Buseghin, Giulio, IT-48100, Ravenna (IT)
(74) Representative: Kraljic, Janez

(57) **Abstract**

Portable inhaler compression device has according to the current invention the option to use energy of eight AA-type standard batteries with an individual nominal voltage of 1.5 V. Such batteries are widely used and may be bought in almost any shop. A special DC/DC inducting converter is used to achieve optimal operation of the mobile inhaler, powered by standard batteries. This diminishes the danger of a patient with a mobile inhaler having no way to receive urgently required inhalations in a critical moment when there is no access to mains power supply, car electrical systems, DC battery or the battery is empty. A portable inhaler compression device according the current invention works efficiently for longer periods, making it possible to perform three full inhalations by powering the electromotor with eight standard AA-type batteries with an individual nominal voltage of 1.5 V. It is assumed that an average inhalation period per single inhalation is 15 minutes.

## Description

The present invention relates to electric portable inhaler compression devices, which also utilize standard batteries, AC electrical power or the vehicle electrical system; the electromotor power guidance relies on a special DC/DC inductive converter.

Piston inhalers, especially piston inhalers used for medical purposes have been is use for quite some time. As examples, we state the following inhalers, as described in the American patents US 2.980.344, US 3.581.742 and US 4.026.285. However, such devices are relatively awkward and heavy. This is the reason why later mobile inhalers were developed for a more convenient use. One such example is stated in patent US 4.244.361. The inhalers described in the patent US 4.244.361, also supports various power sources, among other also batteries.

All known inhalers, which use batteries to power the electromotor, use the power directly from the batteries. Operating times of mobile inhalers, powered by batteries and where the electromotor is directly connected to them, is relatively short, as the battery voltage drops below the minimum required values to power the electromotor fairly soon, which is why the application of battery powered mobile inhalers is severely limited.

The portable inhaler compression devices using a 12 V DC electromotor therefore most frequently use external power sources, such as mains electrical power using a suitable AC/DC converter. Another such external, widely used power source is the electrical power gained from the electrical systems of a car, meaning the car battery or generator. If required, a suitable DC/DC converter is also used. The third type of widely used power source for the electromotor with the mobile inhalers is an added rechargeable battery, which may be attached to the inhaler. Such a DC battery usually consists of 10 rechargeable AA-type batteries with an individual nominal voltage of 1.5 V. The DC battery is usually not included as a standard accessory and must be bought separately.

If a mobile inhaler does not have access to any of the mentioned power sources, i.e. has no access to mains power, vehicle power systems, does not have a DC battery or the battery is empty, it is rendered useless.

The drawback of such mobile inhalers is thusly their inability to use standard batteries to power the electromotor. The mobile inhalers that do use batteries to power the electromotor, however are directly connected to the motor and allow for short and limited operating time, in many cases even less time than is required to execute one single inhalation procedure, which lasts about 15 minutes.

Unlike the currently known mobile inhalers, the portable inhaler compression devices designed after the current invention have the option to use electrical power of eight standard AA-type batteries with an individual nominal voltage of 1,5V. This type of battery is widely available and may be purchased at almost any shop. A special DC/DC inducting converter is used to achieve optimal operation of the mobile inhaler, powered by standard batteries. This diminishes the danger of a patient with a mobile inhaler having no way to receive urgently required inhalations in a critical moment when there is no access to mains power supply, car electrical systems, DC battery or the battery is empty. A portable inhaler compression device designed after the current invention works efficiently for longer periods, making it possible to perform three full inhalations by powering the electromotor with eight standard AA-type batteries with an individual nominal voltage of 1.5 V. It is assumed that an average inhalation period per single inhalation is 15 minutes.

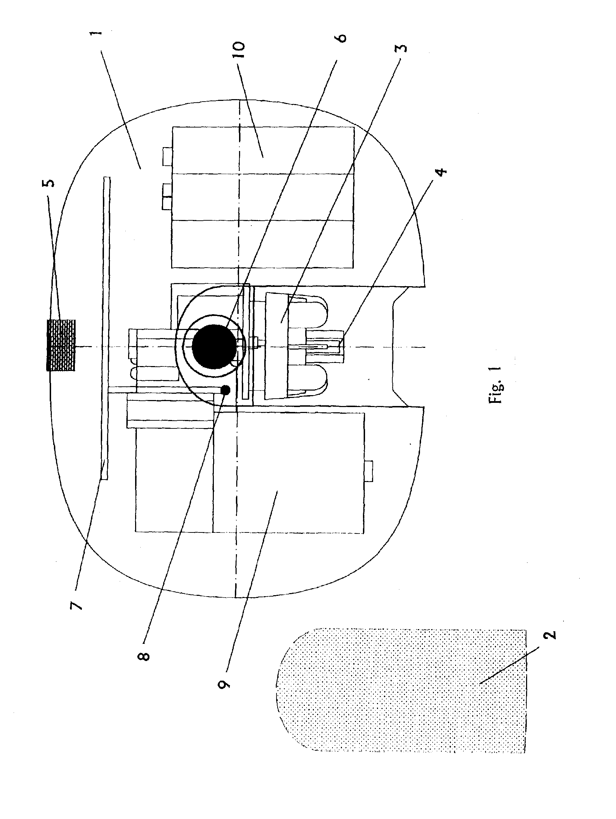

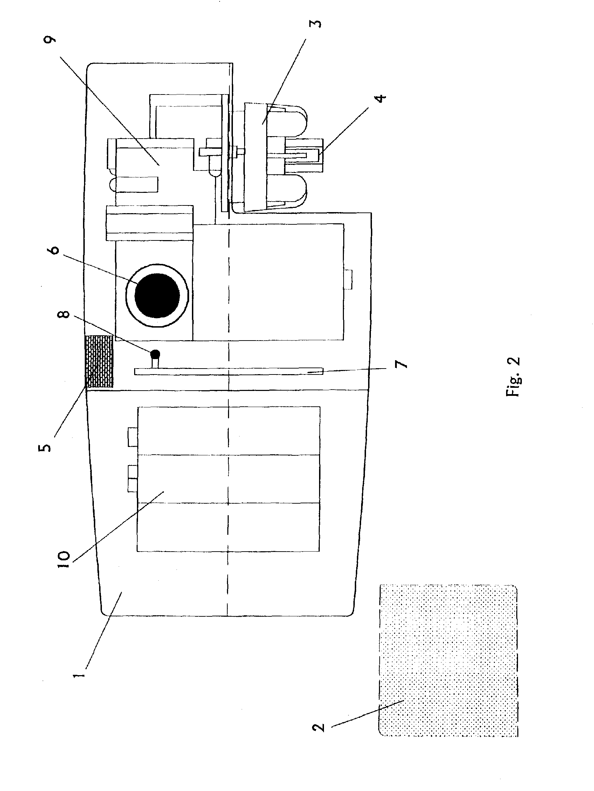

Two model samplers of the portable inhaler compression device utilizing the present invention are shown in figures 1 and 2. The portable inhaler compression device based on the present invention is comprised of the housing or frame (1) of the inhaler, the aesthetic cover (2) of the inhaler, the tightening cover (3) with an air outlet (5), the plug (5) for standard power converters, the ON/OFF power switch (6), the electric circuit of the DC/DC inductive converter (7), signal LED diode (8), 12V DC electromotor with an attached compression device (9) and a cartridge for batteries (10).

In addition to components already mentioned the standard accessories of the portable inhaler compression device also include the appropriate AC/DC converter to get power from the mains supply, DC/DC converter for application in the car and a dispenser complete with a mask and attachment hose to perform the inhalations, which is not indicated on the images.

The core of the portable inhaler compression device is the 12 V DC NICHIBO - HBD5F - C/75 electromotor, which operates at 3600 rotations per minute. The stated electromotor with the attached compression device (9) operates at voltage of 12 V DC electrical and during its operation 700 do 900 mA of DC electric current run through the electromotor. If the electromotor is connected, without the special DC/DC inductive converter, to eight AA-type alkaline batteries with an individual nominal voltage of 1.5 V, we supply the electromotor with the required 12 V DC of voltage. With such a connection, however the voltage of the batteries falls very dramatically and reaches a level below 10.5 V within a few minutes of operation. This voltage is not sufficient for the pump, powered by the electromotor, to produce sufficient working pressure, required for normal inhalation procedure.

To increase the operational time of the portable inhaler compression device with the stated set of eight AA-type standard batteries with an individual nominal voltage of 1,5V, there is a special DC/DC inductive converter designed after the present invention built into the portable inhaler compression device. The stated DC/DC inductive converter (7) is in the electrical connection at the incoming side with the batteries or the battery cartridge (10) (fitting eight AA-type standard batteries with an individual nominal voltage of 1.5 V) and at the outgoing side with the 12 V DC electromotor with the attached compression device (9). The stated DC/DC inductive converter (7) makes it possible for the electric power of the batteries, needed to power the electromotor with its attached compression device (9), to be economically used throughout the operation of the inhaler. The DC/DC inductive converter (7), given the required voltage for the electromotor operation, stabilizes the electric current so that it guarantees an electric current power, greater than 720 mA even after 50 minutes of cumulative operation. At the beginning of operation, after inserting 8 new, unused AA-type standard batteries with an individual nominal voltage of 1.5 V, a current of approximately 900mA runs through the electromotor with the attached compression device (9), which after 50 minutes of cumulative operation drop below the minimum value, which is approximately 720mA, but still sufficient for the compression device to produce satisfactory working pressure for normal inhalation procedure execution.

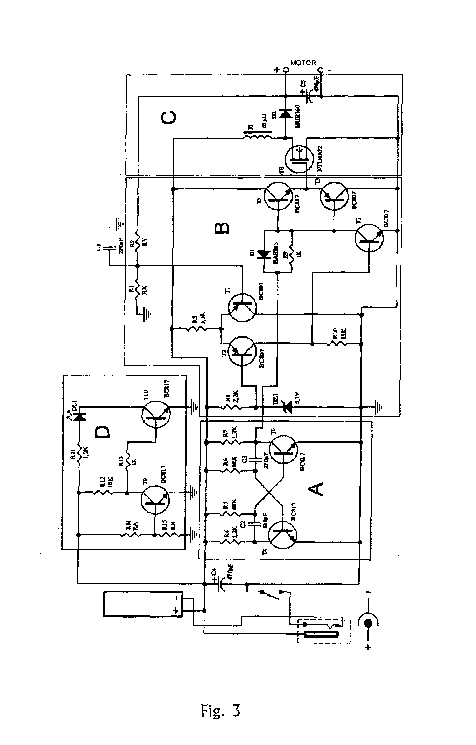

Figure 3 shows the schema of the DC/DC inductive converter circuit (7), which represents a controlled PWM oscillator, comprised of:
- section A, represented by a bi-stable square wave oscillator,
- section B, represented by the work cycle controller,
- section C, represented by the DC/DC upward converter;
- section D, represented by a Smith trigger circuit.

Section D, represented by the Smith trigger circuit, is not critical to the operation of the remainder of the DC/DC inductive converter (7), as it merely sends out a signal when the voltage at the power source connection drops below a predefmed level.

Section A is a simple square wave oscillator, comprised of two bi-polar NPN transistors (T4, T6) and a double RC connection (R4-C2 in R7-C3). The double RC connection defines the frequency of the oscillator, depends on the C2/C3 ratio and governs the work cycle. In the model example, shown in Figure 3, the oscillator frequency is approximately 45 kHz. The example uses a ratio of 330/270, which is approximately 1.22, resulting in a work cycle (ratio between state 1 and state 0 in the square wave) of about 22%. This means that in 100 time units, state 0 will be by 22 time units longer than the half time and state 1 will be by 22 time units shorter than half time. In a cumulative time of 100 units, the work cycle will be in state 0 for 50+22=72 units of time in state 1 for 50-22=28 units of time. This work cycle is defmed with the intent to prevent excessive power supply to section D in any work mode.

Section B is a special circuit, which enables control of work cycle from the square wave oscillator - from section A, in relation to the amount of DC power, brought to the base of the T1 transistor. Switching voltage of the T1 transistor base, as with any bi-polar NPN transistor, is around 700 mV. When the voltage is below 700 mV, the collector-emitter circuit is open, and when the voltage is higher than 700 mV, the circuit collector - emitter is closed. The switching time, needed for the transition from open to closed circuit depends on the characteristics of the chosen T1 transistor (Fo mark). The chosen transistor in this case was Fo approximately 250 MHz, so the switching time was about 4ns. With 4ns of switching time, we can assume that at 45 kHz operation, the switch is immediate, since 4ns at a frequency of 45kHz presents too low a fraction of time to have any kind of influence over the degeneration of the oscillator square wave. The controlling voltage to control the T1 transistor is brought to T1 base directly from the positive voltage connector of the electromotor. The splitting resistors R1/R2 reduce the voltage from the positive connector of the electromotor to a range in close proximity of the switching voltage of the T1 transistor (approximately 700 mV). The C1 capacitor is used to stabilize the voltage, thus preventing unstable control of the T1 transistor base, which would trigger auto-oscillation. When the control voltage at T1 base is below 700mA, the emitor collector circuit in the T1 transistor is open. T2 transistor, together with the DZ1 zener diode and the R8 resistor form a simple fixed voltage generator. When the emitor collector circuit in T1 transistor is open, i.e. when the voltage on the positive connector of the electromotor is too low, the voltage at T2 collector is approximately 1000mV. At this condition the T7 transistor is short-circuited with mass and the T3 transistors (bi-polar NPN transistor) is open and T5 (bi-polar PNP transistor) is closed. So the MOSFET T8 transistor port will receive the control voltage in 28 cycles of the setion A oscillator. When the voltage at the positive connection of the electromotor is within the specified working range, a reverse situation occurs, and the circuit emitor collector of the T1 transistor closes. The number of cycles on the MOSFET T8 transistor will reduce from the former 28 cycles from section A and will serve to control the work cycle of the oscillator in section A. The number of state 0 will increase and the number of state 1 will reduce, which results in a more economic use of the current from the power source, in this case the batteries. Such controlling is done up to 10.000 times per second. The electric current from the batteries is drawn in surges and not continually, however they represent a sufficiently powerful source of electric current, so that the electromotor can power the compression device sufficiently to perform the inhalation procedure.

Section C is a classing DC/DC upward converter, based on the energy stored in J1 coil, which is which is use on the user, in our case the electromotor, through an ultra-fast D2 directional diode. When the MOSFET T8 transistor is closed, the J1 coil stores a large amount of energy and when T8 is open, the energy, stored in J1, is transfered to the electromotor using the D2 directional diode. Section C, if it were not controlled by section B, would rapidly empty the batteries to power the electromotor.

Section B controls section C so that, based on need, in decided how much current is to be transfered to the electromotor, so that the pump, connected to it will produce enough pressure to execute the inhalation procedure. Put in another way, section B controls section C, by letting through such amount of current, required to maintain sufficient power of the electromotor, so that the pump, connectied to it, will maintain sufficient pressure to perform the inhalation process. This way we can control the flow of current from the batteries very precisely and only allow the minimum flow required for the sufficient electromotor power, to create the sufficent pressure in the compression device for the inhalation process.

Table 1 specifies measured value for electrical voltage at the electromotor DC connections, with the attached compression device (9), which is connected to the power cource (battery cartridge(10)) via DC/DC inductive converter (7), the measured values of starting and ending pressure, achieved by the pump, which is attached to the DC electromotor, at the start and end of test inhalation procedures, lasting 154 minutes each.

| Duration of operation | Starting pressure (mbar) | Ending pressure (mbar) | Voltage at the end (V DC) |
|---|---|---|---|
| 0 - 15 min | 716 | 610 | 11,88 |
| 16 - 30 min | 671 | 576 | 11,29 |
| 31 - 45 min | 600 | 500 | 9,65 |
| 46 min | 507 | 490 | 8,85 |
| 47 min | / | 480 | 8,84 |
| 48 min | / | 480 | 4,77 |
| 49 min | / | 470 | / |
| 50 min | / | 460 | / |
| | | | |

The test measurements are based on three consequtive simulated inhalation procedures, each lasting on average 15 minutes. After the three simulated procedures were completed, measurement values were taken for another five minutes. After five minutes the pressure produced drops below the minimum level required to perfom a normal inhalation procedure. The measurement values show that the portable inhaler compression device augmented by the invention, using a special DC/DC inductive converter (7), is able to deliver three full inhalation procedures using a single set of eight AA-type standard batteries witn an individual nominal voltage of 1.5 V DC.

The portable inhaler compression device designed after the current invention can, using a special plug (5), operate also by means of an external 12 V DC power source. When powering the electromotor with the pump (9) using any other external source of electric energy, such as the mains supply, using a special AC/DC converter or supplying energy from a vehicle power system, using a special DC/DC converter, the connection to the battery cartridge (10) will be terminated proptly.

SW1 mono-pole switch serves to activate or deactivate the DC electromotor with the attached pump (9) and consequently the portable inhaler compression device.

The dimensions of the DC/DC inductive converter with the circuitry and all elements included are 48.9 mm x 34.3 mm x 15 mm.

## Claims

1. A portable inhaler compressor device, comprised of the housing or frame (1), aesthetic cover (2) of the inhaler, tightening cover (3) with an air inlet (4), ON/OFF switch (6), 12 V DC electromotor with the attached compression device (9), **characterized in that** in order to supply electric power to the electromotor, to which a compression device(9) is attached, it can use standard batteries, which are connected via a DC/DC inductive converter (7) to the 12 V DC electromotor with the attached compression device (9).

2. A portable inhaler compressor device according to claim 1, **characterized in that** it has a built-in plug (5) for external 12 V DC electric current supply.

3. A portable inhaler compressor device according to a previous claim, **characterized in that** the stated DC/DC inductive converter (7) is in the form of a controlled PWM oscillator, comprised of:
- section A, represented by a bi-stable square wave oscillator,
- section B, represented by the work cycle controller,
- section C, represented by the DC/DC upward converter;
where section B control section C **in that** it maintains the amount of minimum current, required to power the electromotor sufficiently, thus enabling the compression device, attached to it, is able to attain sufficient pressure to perform the inhalation process.

4. Portable inhaler compression device according to a previous claim, **characterized in that** the stated DC/DX inductive converter (7) includes the additional
- section D, which represents a Smith trigger circuit, giving a signal, when the incoming voltage at the connection of the power source fall below a given level.
